# EUROPEAN PATENT SPECIFICATION

(11) **EP 0 636 599 B1**
(45) Date of publication and mention of the grant of the patent: **14.06.2000**
(21) Application number: 94100795.7
(22) Date of filing: 20.01.1994
(51) Int. Cl.: C07C 53/08, C07C 51/12, C07C 53/12, C07C 51/56, C07C 69/14, C07C 67/36, C10K 1/00, B01D 53/34

(54) **Process for the production of acetic acid**
Verfahren zur Herstellung von Essigsäure
Procédé pour la préparation d'acide acétique

(30) Priority: 27.07.1993 KR 9314265
(43) Date of publication of application: 01.02.1995
(73) Proprietor: KOREA INSTITUTE OF SCIENCE AND TECHNOLOGY, Seoul (KR)
(72) Inventor: Uhm, Sung Jin, Kangnam-ku, Seoul (KR); Han, Sung Hwan, Bundang-ku, Seongnam-shi, Kyonggi-do (KR); Oh, Jun Woo, Dobong-ku, Seoul (KR); Joo, Oh Shim, Seongbuk-ku, Seoul (KR)
(74) Representative: Turi, Michael, Dipl.-Phys.

(56) References cited:
- EP-A- 0 335 625
- EP-A- 0 350 635
- GB-A- 247 050
- US-A- 3 717 670

## Description

The present invention relates to a process for selectively preparing acetic acid by carbonylating methanol with carbon monoxide.

### BACKGROUND OF THE INVENTION

Acetic acid and acetic anhydride have long been used as basic chemicals for industrial purposes such as solvents, raw materials and intermeidates for various reaction products.

Acetic acid has been traditionally produced by oxidation of ethanol or acetaldehyde prepared by Wacker process starting from ethylene or low boiling raffinates produced in a petroleum process. These oxidation processes are being replaced by a carbonylation process which reacts methanol with carbon monoxide employing a rhodium catalyst in the presence of methyl iodide in liquid phase. However, the liquid phase carbonylation process has been found to have various critical deficiencies including the continuous loss of the expensive catalyst and the high corrosion problem stemming from the liquid phase reaction mixture which entail extremely high construction, maintenance and production costs.

In order to overcome the various problems associated with the liquid phase carbonylation of methanol to produce acetic acid, therefore, various proposals have been made to provide a process for producing acetic acid in a gas phase. For example, the processes disclosed in European Patent Publication No. 0 069 514 A2 assigned to Toyo Engineering Corporation, German DE 33 23 654, and Ind. Chem. Prod., Res. Dev., 22, 436(1983) and Chemistry Letters, 895(1987) relate to the gas phase production of acetic acid by a nickel-catalyzed carbonylation of methanol; however, none of these processes has proven to be commercially viable due to various problems.

European Patent Publication No. 0 335 625 A2 provides a process for producing acetic acid by employing a nickel/rhodium catalyst supported on active carbon at 188°C. In this process, a mixture of CO/H₂(1:2) gas is introduced under a pressure of 9 atm, with the ratio of methanol to methyl iodide being 100:19.1 and the LHSV of the feed being 1. However, this process results in a low yield of 9.7%. In addition, nickel is apt to be vaporized from the catalyst beds during the reaction, thereby shortening the life time of the catalyst.

U.S. Patent Nos. 3,717,670(to Hockman) and 3,689,533 (to Schultz) offer processes for producing acetic acid in a heterogeneous gas phase using a rhodium catalyst. These patents teach that the conversion of methanol and the yield of acetic acid may be improved by mixing the Rh catalyst with a metallic component. However, according to these patents, the methanol conversion, the selectivity and the yield of acetic acid are no more than 78.5%, 58% and 45.5%, respectively, under the most preferred reaction conditions: i.e., a reaction temperature of 285°C and pressure of 200 psi, with the molar ratio of CH₃I:CH₃OH:CO being 1:12.3:26.2.

Japanese Laid-open Patent Publication No. Sho 48-80511 describes a gas phase process for preparing acetic acid wherein a rhodium compound is employed as a catalyst and a small amount of cobalt, nickel or iron salt and/or aluminum, copper, titanium, mercury or lithium salt is added as a co-catalyst. In this method, methanol, carbon monoxide and methyl iodide are introduced at a rate of 169 g/hr, 224 g/hr and 27 g/hr, respectively, using the catalyst prepared by supporting 0.43 g of RhCl₃·4H₂O, 0.43 g of NiCl₂, 0.44 g of AlCl₃ and 0.43 g of LiCl on 25 g of active carbon; and the reaction is carried out at 230°C under 220 psi. However, this process gives an acetic acid yield of 71%.

U.S. Patent No. 4,918,218 to Mueller, et al. and German Patent No. 36 06 169 relate to a gas phase process using a nickel/palladium complex catalyst system and a process using a cobalt catalyst supported on zeolite, respectively. However, neither process has been regarded as commercially viable in view of their low reactivity, conversion and selectivity to acetic acid.

As a separate but related matter, among the various problems that exist in the afore-mentioned gas phase carbonylation processes to produce acetic acid, the most critical impediment to their commercialization has been the short life time of the expensive(e.g., rhodium) catalyst due to its contamination by impurities present in the feed gas, i.e., CO.

As a matter of fact, the task of dealing with the contamination of catalysts is a pervasive one throughout the chemical industry as numerous chemicals are prepared by catalytic reactions using an industrial gas such as synthesis gas(CO/H₂) and carbon monoxide. Representative of such reactions include hydroformylation and carbonylation of various reactants to produce, e.g., acetic acid and acetic anhydride, as discussed above. In these reactions, expensive noble metals, including rhodium, are generally used as a catalyst.

The afore-mentioned industrial gases can be manufactured by various known processes. During the processes, various impurities, especially iron carbonyl compounds, are formed as the gases come in contact with iron. Also, when they are stored in an iron vessel at a room temperature for a substantial period of time, a significant amount of iron carbonyl compounds may be formed.

Said iron carbonyl compounds have been found to cause serious problems in carrying out the above catalytic reactions as they tend to accumulate on the active surface of the catalyst and poison the catalyst rapidly. The iron carbonyl compounds, even in a minor amount, may degrade the catalyst performance, including its reactivity and selectivity, after a repeated use thereof. Accordingly, unless and until a commercially feasible solution is found to remove the catalyst contamination problem, there may be no practicable alternative to, e.g., the existing liquid phase process for the production of acetic acid discussed above.

GB 247,050 discloses the purification of gases, e.g. water gas, from iron carbonyl by means of a chemically acting component, e.g. oxygen, chlorine, hydrogen chloride and phosgene. Iodine is not mentioned in this patent.

### SUMMARY OF THE INVENTION

Accordingly, it is an object of the invention to provide a process for selectively producing acetic acid by the gas phase carbonylation of methanol with carbon monoxide, said carbon monoxide optionally in admixture of hydrogen, using a Rh catalyst and a halide co-catalyst wherein all or substantially all of the methyl acetate separated from the production mixture together with the co-catalyst is recycled.

It is a still further object of the invention to provide a novel method for sustaining the active life time of a carbonylation or a hydroformylation catalyst by pretreating the carbon monoxide or the synthesis gas to be used in carrying out the gas phase carbonylation or the hydroformylation.

In accordance with the present invention, there is provided a gas phase process for the selective production of acetic acid, which comprises:
a) pretreating carbon monoxide containing metallic carbonyl compounds by feeding it into a purification column wherein iodine is introduced in a molar amount ranging from 0.1 to 1000 times the metallic content in the feed gas and which is maintained at a temperature to allow the metallic carbonyl impurities to react completely with the iodine gas, sending the metallic iodides formed to an adsorbent column and allowing the metallic iodides to be adsorbed onto the adsorbent therein;
b) carbonylating methanol in a gas phase with said pretreated carbon monoxide in a carbonylation reactor, said carbon monoxide being optionally in admixture of hydrogen, in the presence of a rhodium catalyst comprised of a rhodium compound and a second metallic component selected from the group consisting of an alkali metal, an alkaline earth metal, Co and a mixture thereof, and supported on an inert material, and a halide co-catalyst at a temperature ranging from 200°C to 280°C, a pressure ranging from 10 to 20 atm and a methanol GHSV ranging from 300 to 5000 hr⁻¹ to provide a product mixture composed of acetic acid in a major amount and methyl acetate in a minor amount;
c) separating said methyl acetate in the minor amount and the co-catalyst from the product mixture and recycling the separated methyl acetate and the co-catalyst to the carbonylation reactor;and
d) recovering from the product mixture said acetic acid in the major amount.

### DETAILED DESCRIPTION OF THE PREFERRED EMBODIMENTS

### A. Pretreatment of Carbon Monoxide or Syn Gas

As previously stated, an industrial gas, i.e., carbon monoxide gas or syn gas, may contain as impurities metallic (e.g., iron) carbonyl compounds. These impurities tend to contaminate or poison the catalyst employed in an Oxo process or a carbonylation process rather rapidly, rendering the process commercially ineffective.

In accordance with the present invention, it has been found that such catalyst contamination problem can be effectively removed by treating the syn gas or carbon monoxide, prior to its exposure to the hydroformylation or carbonylation catalyst, with iodine gas.

Specifically, an industrial gas, e.g., CO, containing metallic carbonyl compounds, is fed into a purification column wherein iodine is introduced. The amount of iodine to be used is determined as a function of the flow rate of the feed gas and the column temperature. Said iodine is introduced into the column in a molar amount ranging from 0.1 to 1000, more preferably from 0.5 to 100, and, most preferably, from 1 to 10 times the metallic content in the feed gas. Several trays may be installed in the column to ensure good mixing between the feed gas and the iodine. The column is maintained at a temperature, for example, in a range from 150°C to 200°C, to allow the metallic carbonyl impurities to react completely with the halogen gas. The metallic halides, e.g., iron and/or nikel iodide, are sent to an adsorption column and adsorbed onto the adsorbent therein, producing the desired purified feed gas. Examples of the adsorbent which may be used in the present invention include active carbon, clay, alumina, silica, silica-alumina, zeolite and other adsorbents commonly used in the art. The feed gas thus purified is transferred to, e.g., a carbonylation reactor.

The industrial gas treated in accordance with the present invention may contain, if any, the metallic carbonyl compounds only in a negligible amount, e.g., 1 ppb or less, which is rather difficult to detect by a conventional analysis method. Consequently, the problem of the catalyst poisoning and deactivation encountered in an Oxo or a carbonylation process can be effectively resolved.

### B. Selective Production of Acetic Acid and/or Methyl Acetate

In accordance with the present invention, a process for the selective production of acetic acid and/or methyl acetate is accomplished by controlling the reaction conditions in the gas phase carbonylation of methanol with carbon monoxide in the presence of a rhodium catalyst and a halide co-catalyst(which is sometimes called a promoter).

Said carbon monoxide is pretreated with iodine gas as described previously; and, if desired, may be introduced together with an appropriate amount of hydrogen gas to enhance the selectivity and yield of acetic acid or methyl acetate.

The carbon monoxide may be introduced at a pressure near or slightly higher than the reaction pressure, e.g., 13 atm and at a temperature preheated to a desired reaction temperature, e.g., 250°C; and employed in a molar ratio of methanol to carbon monoxide ranging from 1:0.1 to 1:100, more preferably 1:0.5 to 1:50, most preferably 1:0.8 to 1:3.

Similarly, methanol is preferably preheated and vaporized to the desired reaction temperature and pressure prior to its introduction into the reaction system.

### B.1. Selective production of acetic acid

In accordance with the present invention, when it is desired to produce acetic acid primarily, the selectivity to acetic acid is enhanced by adjusting the reaction conditions: i.e., the reaction temperature to range from room temperature to 500°C, more preferably from 150 to 300°C, most preferably from 200 to 280°C; the reaction pressure to range from atmospheric pressure to 300 atm, more preferably from 5 to 30 atm, most preferably from 10 to 20 atm; and the contact time of the reactants with the catalyst as inversely represented by the GHSV of methanol to range from 1 to 100,000 hr⁻¹, more preferably from 100 to 10,000 hr⁻¹, and most preferably from 300 to 5,000 hr⁻¹.

In accordance with a preferred embodiment of the present invention, it has been found that the selectivity to acetic acid can be maintained at a level of 99% or higher by initially adding an appropriate amount of methyl acetate (e.g., about 10 mol% based on the methanol used) to the feed stream of methanol, and, thereafter, separating and recycling the methyl acetate together with the co-catalyst, e.g., methyl iodide, used.

In accordance with another preferred embodiment of the present invention, hydrogen may be beneficially added in an amount up to 50% by volume to the feed gas, i.e., carbon monoxide. In the case of conventional liquid phase reactions, when hydrogen is present in carbon monoxide, various side reactions may occur, thereby lowering the reaction efficiency. However, in the gas phase catalytic reaction of the present invention, the presence of hydrogen in the reaction system in fact considerably enhances the conversion of methanol and selectivity to acetic acid(or methyl acetate depending on the reaction conditions employed). Also, the ability to use a mixture of hydrogen and carbon monoxide has an added advantage since it obviates the need to prepare carbon monoxide free from hydrogen.

In a further preferred embodiment of the present invention, water may be added in the reaction system, particularly to the recycling stream of the methyl acetate, up to 20 mol% based on the methanol used, in order to improve the selectivity to acetic acid. However, if a higher yield of methyl acetate is desired, the water content in the reactants should be maintained at a lowest possible level so as to inhibit the formation of acetic acid.

### B.3. Catalyst system

The rhodium catalyst for use in carrying out the gas phase carbonylation of methanol in accordance with the present invention comprises a rhodium compound and a second metallic component selected from the group consisting of an alkali metal, an alkaline earth metal, a transition metal and a mixture thereof; and may be prepared by depositing or impregnating a solution of the rhodium compound dissolved in water or an organic solvent, e.g., an alcohol, on an inert supporting material together with the second metallic compound, calcining the resultant at a temperature ranging from 200 to 500°C. The inert supporting material which may be used in preparing the catalyst includes active carbon, clay, alumina, silica, silica-alumina, alumina-phosphate, alumina-silica-phosphate, magnesia, zirconia and the like.

Any of the rhodium compounds, which are soluble in water or an organic solvent and can be calcined at the temperature range of 200 to 500°C, may be used. Representative of such rhodium compounds are: RhX₃, RhX₃·3H₂O, Rh₂(CO)₄X₂, [Rh(CO)X₄]Y, Rh₂(CO)₈, Rh(NO₃)₃, [Rh(CO)₂X₂]Y, Rh₂O₃, Rh(CH₃COO)₃, [Rh(C₂H₄)₂X]₂, Rh[(C₆H₅)₃P]₂(CO)X, Rh metal, RhX[(C₆H₅)₃P]₂(CH₃X)₂, Rh(SnX₃)[(C₆H₅)P]₃, RhX(CO)[(C₆H₅)₃Q]₂, (R₄Z)[Rh(CO)₂X]₂, (R₄Z)₂[Rh(CO)X₄], RhX[(C₆H₅)₃P]₃, RhX[(C₆H₅)₃P]H₂, [(C₆H₅)₃P]₃Rh(CO)H and Y₄Rh₂X₂(SnX₃)₄ wherein X is Cl, Br or I; Y is Na, Li or K; Z is N, As or P; Q is As, P or Sb; and R is a C₁ to C₁₂ alkyl or aryl group. Preferably, RhCl₃·3H₂O or Rh(NO₃) is used.

The rhodium compound may be employed in an amount of 0.01 to 20% by weight, more preferably 0.1 to 10%, most preferably 0.3 to 5% by weight of Rh based on the amount of the supporting material. The transition metal compound may be added in an amount of 1 to 1000 mol%, more preferably 10 to 500 mol%, most preferably 30 to 300 mol%, based on the amount of rhodium. The alkali metal or the alkaline earth metal compound may be added in an amount of 1 to 2,000 mol%, more preferably 50 to 1000 mol%, most preferably 200 to 800 mol%, based on the amount of rhodium.

The alkali metal which may be employed as the second component in the rhodium catalyst includes Li, Na, K, Rb, Cs and Fr.

The alkaline earth metal which may be employed as the second component includes Be, Mg, Ca, Sr, Ba and Ra.

The transition metal which may be employed as the second component is Co.

The carbonylation catalyst according to the present invention is easily prepared by adding at least one of the second metallic compounds such as CoCl₂, LiI, NaI, KI, RbCl, BeCl₂, MgCl₂, CaCl₂, SrCl₂, BaCl₂ and the like in a specified amount to a rhodium compound supported on the inert material.

The inventive gas phase process for selectively producing acetic acid and/or methyl acetate is carried out by using a halide co-catalyst in the presence of the rhodium catalyst.

The halide compound which may be employed as the co-catalyst includes: CH₃I, CH₃Br, CH₃Cl, I₂, Br₂, Cl₂, HI, HBr, HCl and the like. Among them, CH₃I is preferred.

The halide co-catalyst may be employed in a molar ratio of the co-catalyt per mole of the methanol used ranging from 0.001 to 5, more preferably from 0.01 to 1 and most preferably from 0.05 to 0.15.

### Example 1

Carbon monoxide maintained at a room temperature and the pressure of 100 atm containing approximately 1 ppm of iron carbonyl compounds, predominantly in the form of Fe(CO)₅, was treated with iodine at a concentration of approximately 5 ppm in a purification column. About 10 m³ of the gas so treated was passed through an adsorption column which was filled with active carbon and maintained at 200°C. Under a standard temperature and pressure, carbon monoxide(10m³) thus treated was collected and then passed through a gas scrubber containing 1L of conc. sulfuric acid to dissolve the remaining iron carbonyl compounds in the gas after the treatment. Concentration of the iron ion in the sulfuric acid was measured to be 0.01 ppm or less.

It should be noted that carbon monoxide employed in all of the following Examples given in this specification is pretreated with iodine in a manner similar to the procedure described in Example 1 above, unless specified otherwise.

### Example 2

RhCl₃ and LiI were supported on active carbon by impregnating a solution of RhCl₃ and LiI on the carbon such that 0.6% by weight of Rh based on the amount of the active carbon and 400mol% of LiI based on the amount of Rh were supported thereon. The resulting material was then calcined at 300°C to prepare the catalyst.

A reactor tube, made of titanium and having an inside diameter of 1.27 cm(0.5 inch) and a length of 40 cm, was charged with 5g of the catalyst. The reactor tube was filled with glass fiber, pretreated in a NaOH solution, at the top and the bottom ends thereof so as to form a catalyst bed of 10 cm in length therein; and, a thermowell having an outside diameter of 0.64 cm(0.25 inch) with a thermocouple was inserted in the center of the reactor tube. The reactor tube was oil jacketed so as to heat it with a heating medium. Methanol and carbon monoxide in a molar ratio of 1:2.3 were introduced into the reactor tube; and were allowed to react in the presence of 10 mol% of the co-catalyst, CH₃I, based on the amount of the methanol used at an inside temperature of about 240°C under a pressure of 200 psi.

The conversion of methanol, and the yields of acetic acid and methyl acetate depending on the GHSV of methanol under the above conditions are shown in Table 1 below.

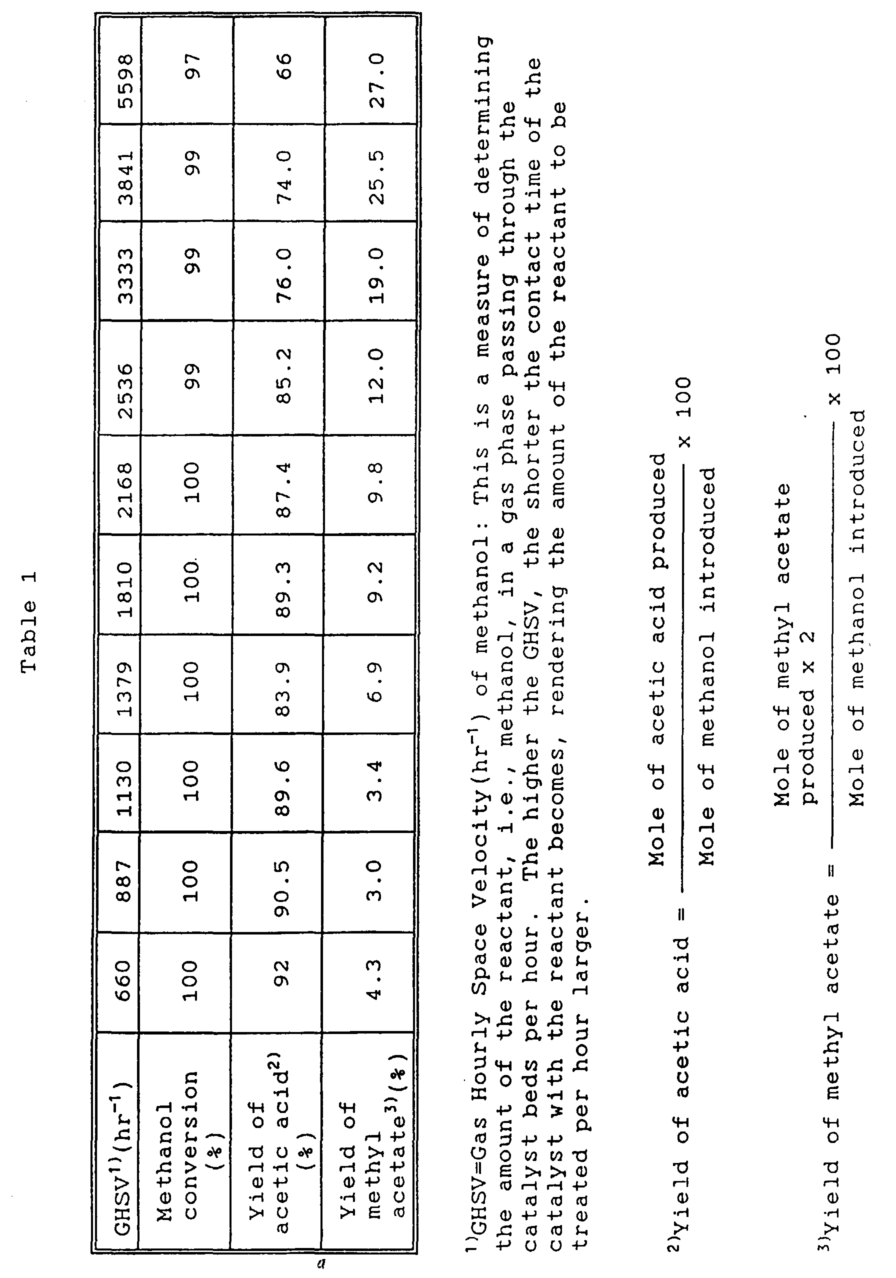

### Example 3

This Example was carried out in accordance with the procedure as described in Example 2, except that two separate feed streams of carbon monoxide were employed: one of the streams had been treated by passing it through a column provided with iodine gas followed by an adsorption column packed with silica; and the other stream had not been so treated. After conducting the carbonylation by employing each of the non-treated (Experiment 1) and pretreated (Experiment 2) carbon monoxide gases for given periods, the yields of the products were measured at the GHSV of methanol of 600hr⁻¹ and 5000hr⁻¹, respectively. The results are shown in Table 2.

**Table 2**

| Experiment | Period | Methanol conversion (%) | Yield of acetic acid(%) | Yield of methyl acetate(%) |
|---|---|---|---|---|
| 1 | 70days, at the GHSV of 600 | 80.3 | 33.1 | 57.2 |
| 2 | 75days, at the GHSV of 5000 | 95.3 | 78.2 | 17.1 |

As can be seen from Table 2, when the carbonylating gas, i.e., carbon monoxide, is pretreated with iodine gas, the activity of the catalyst is maintained even after its using for a substantially long period of time.

### Example 4 (for comparison)

This Example was carried out in accordance with the procedure as described in Example 2, except that the reaction temperature and pressure were changed to 233½C and 150 psi, respectively, in addition to the different values of GHSV. The results are shown in Table 3 below.

**Table 3**

| GHSV(hr⁻¹) | 1207 | 1568 | 1735 | 2133 | 3293 | 3652 | 4149 |
|---|---|---|---|---|---|---|---|
| Methanol conversion (%) | 96.4 | 93.7 | 88.9 | 84.3 | 71.6 | 68.8 | 62.2 |
| Yield of acetic acid(%) | 33.0 | 22.3 | 20.0 | 15.5 | 10.7 | 8.6 | 7.5 |
| Yield of methyl acetate(%) | 63.0 | 64.8 | 64.8 | 63.6 | 60.1 | 54.5 | 49.5 |

As can be seen from Tables 1 and 3, even if an identical catalyst is used, the production ratio of methyl acetate to acetic acid can easily be adjusted under different reaction conditions. That is, the results from Example 4 show that methyl acetate can be produced in a higher selectivity and yield by adjusting the reaction conditions, i.e., by lowering the reaction temperature and pressure and shortening the contact time of the methanol with the catalyst.

### Example 5

This Example was carried out in accordance with the procedure as described in Example 2, except that a catalyst prepared by supporting 800mol% of LiI on active carbon, with different GHSV values, was employed. The results are shown in Table 4 below.

**Table 4**

| GHSV(hr⁻¹) | 600 | 900 | 1200 | 1500 | 1800 | 2100 | 2400 | 2700 |
|---|---|---|---|---|---|---|---|---|
| Methanol conversion (%) | 100 | 100 | 100 | 100 | 100 | 100 | >99 | >99 |
| Yield of acetic acid(%) | 91.5 | 92.1 | 93.6 | 92.7 | 92.2 | 92.6 | 78.5 | 78.9 |
| Yield of methyl acetate(%) | 6.5 | 6.3 | 5.1 | 6.0 | 5.6 | 6.1 | 19.2 | 19.8 |

### Example 6

This Example was carried out as described in Example 2, except that a catalyst supported on active carbon and containing 1.8% by weight of Rh based on the amount of active carbon and 400 mol% of LiI based on the amount of Rh was employed, and the reaction temperature and the pressure were changed to 270°C and 150 psi, respectively, in addition to the different values of GHSV. The results are shown in Table 5 below.

**Table 5**

| GHSV(hr⁻¹) | 1000 | 2000 | 3500 | 4800 |
|---|---|---|---|---|
| Methanol conversion (%) | 99.8 | 99.9 | 100 | 99.8 |
| Yield of acetic acid(%) | 94.8 | 83.3 | 75.1 | 68.6 |
| Yield of methyl acetate(%) | 5.0 | 16.1 | 22.5 | 30.2 |

### Example 7

This Example was carried out as described in Example 2, except that a catalyst supported on active carbon and containing 0.6% by weight of Rh based on the amount of the active carbon and 200 mol% of NaI based on the amount of Rh was employed, and the reaction temperature and the pressure were changed to 240°C and 200 psi, respectively, in addition to the different values of GHSV. The results are shown in Table 6 below.

**Table 6**

| GHSV(hr⁻¹) | 1000 | 1568 | 1735 | 2133 |
|---|---|---|---|---|
| Methanol conversion (%) | 100 | 99.9 | 100 | 99.7 |
| Yield of acetic acid(%) | 82.0 | 57.4 | 38.8 | 31.1 |
| Yield of methyl acetate(%) | 17.1 | 42.1 | 60.0 | 67.9 |

### Example 8

This Example was carried out in the same manner as described in Example 2, except that a catalyst supported on active carbon and containing 0.6% by weight of Rh based on the amount of the active carbon and 200 mol% of KI based on the amount of Rh, together with the different values of GHSV, was employed. The results are shown in Table 7 below.

**Table 7**

| GHSV(hr⁻¹) | 1039 | 1795 | 2997 | 4017 |
|---|---|---|---|---|
| Methanol conversion (%) | 100 | 100 | 99.7 | 96 |
| Yield of acetic acid(%) | 94.8 | 80.0 | 50.1 | 30.1 |
| Yield of methyl acetate(%) | 5.1 | 19.9 | 48.9 | 60.5 |

### Example 9

This Example was carried out as described in Example 2, except that a catalyst supported on active carbon and containing 0.6% by weight of Rh based on the active carbon and 50 mol% of MgCl₂ based on the Rh, together with the different values of GHSV, was employed. The results are shown in Table 8 below.

**Table 8**

| GHSV(hr⁻¹) | 2068 | 3417 | 4855 | 5754 |
|---|---|---|---|---|
| Methanol conversion (%) | 100 | 98.8 | 94.6 | 84.9 |
| Yield of acetic acid(%) | 89.9 | 66.6 | 43.1 | 30.0 |
| Yield of methyl acetate(%) | 8.8 | 30.6 | 49.6 | 52.3 |

### Example 10

This Example was carried out as described in Example 2, except that a catalyst supported on active carbon and containing 0.6% by weight of Rh based on the active carbon and 50 mol% of CoCl₂ based on the Rh was employed; and the reaction temperature was changed to 210°C, in addition to the changed values of GHSV. The results are shown in Table 9 below.

**Table 9**

| GHSV(hr⁻¹) | 1000 | 2000 | 3000 | 4000 |
|---|---|---|---|---|
| Methanol conversion (%) | 99.9 | 98.0 | 91.1 | 82.3 |
| Yield of acetic acid(%) | 45.0 | 32.3 | 19.9 | 12.3 |
| Yield of methyl acetate(%) | 53.8 | 64.6 | 70.1 | 68.9 |

### Example 11

This Example was carried out employing the same catalyst as described in Example 2, except that a certain amount of hydrogen was mixed with the carbon monoxide.

While changing the ratio of carbon monoxide to hydrogen, the mixed gas of carbon monoxide and hydrogen was introduced together with methanol into the reactor under a pressure of 14.1 kg/cm²(200 psi). At this time, the molar ratio of methanol to carbon monoxide was 1:1.6, the GHSV of methanol was maintained at 1500 hr⁻¹ at the reaction temperature of 250°C. The results are shown in Table 10 below.

**Table 10**

| | CO only | 7%(by volume) of H₂ added | 14% of H₂ added | 20% of H₂ added | 25% of H₂ added |
|---|---|---|---|---|---|
| Methanol conversion (%) | 99.9 | 99.8 | 100 | 99.9 | 100 |
| Yield of acetic acid(%) | 85.1 | 97.3 | 95.1 | 92.9 | 92.1 |

As can be seen from Table 10, when a certain amount of hydrogen is mixed with the carbon monoxide under certain reaction conditions, the yield of acetic acid can be enhanced.

### Example 12

RhCl₃·3H₂O and LiI were supported on active carbon in an aqueous phase in such a manner that 0.6% by weight of Rh based on the amount of the active carbon and 200mol% of LiI based on the amount of Rh were contained thereon. The resultant was then calcined at 300°C to prepare the catalyst.

Using the catalyst obtained, this Example was carried out as described in Example 11 except that the GHSV of methanol was maintained at 3000 hr⁻¹. The results are shown in Table 11 below.

**Table 11**

| | CO only | 7% of H₂ added |
|---|---|---|
| Methanol conversion(%) | 86.2 | 100 |
| Yield of acetic acid(%) | 21.6 | 65.9 |
| Yield of methyl acetate(%) | 64.4 | 31.0 |

### Example 13

This Example was carried out as described in Example 11, except that a catalyst supported on active carbon and containing 0.6% by weight of Rh based on the active carbon and 200mol% of KCl based on the Rh was employed, and the GHSV of methanol was maintained at 2000hr⁻¹. The results are shown in Table 12 below.

**Table 12**

| | CO only | 7% of H₂ added |
|---|---|---|
| Methanol conversion(%) | 86.9 | 100 |
| Yield of acetic acid(%) | 22.5 | 46.1 |
| Yield of methyl acetate(%) | 60.4 | 50.1 |

### Example 14

This Example was carried out as described in Example 2, except that a catalyst supported on active carbon and containing 1.8% by weight of Rh based on the active carbon and 400 mol% of LiI based on the Rh was employed; the reaction temperature and the pressure were changed to 250°C and 200 psi, respectively; and the GHSV of methanol was maintained at 1800 hr⁻¹, while adding various amounts of methyl acetate to the feed stream of methanol. The results are shown in Table 12.

**Table 13**

| Amount of methyl acetate added(mol%) | 0 | 5* | 10* |
|---|---|---|---|
| Methanol converision (%) | 100 | 100 | 100 |
| Yield of acetic acid(%) | 80.2 | 93.4 | 99.9 |
| Yield of methyl acetate (%) | 18.8 | 16.0 | 20.1 |
| Total | 100 | 110* | 120* |

| | | | |
|---|---|---|---|
| * 1 mol% of methyl acetate added to the reaction system is counted as 2 mol% of methyl acetate as yielded from the reaction system in accordance with the second formula given on page 28 hereof. The same holds true with respect to the data shown in Tables 23 to 26 hereof. | | | |

As can be seen from Table 13, when methanol is introduced together with an appropriate amount of methyl acetate, the yield of acetic acid can be enhanced significantly. Accordingly, if an appropriate amount of the methyl acetate produced by the carbonylation is recycled to the carbonylation reactor, the yield of acetic acid can be increased substantially.

### Example 15

This Example was carried out as described in Example 14 except that various amounts of water were added to the methanol feed containing 10 mol% of methyl acetate. The results are shown in Table 26.

**Table 14**

| Amount of water (mol%) | 0 | 10 | 20 |
|---|---|---|---|
| Methanol conversion (%) | 99.9 | 100 | 100 |
| Yield of acetic acid(%) | 99.8 | 111.9 | 114.3 |
| Yield of methyl acetate(%) | 19.9 | 8.1 | 5.7 |
| Total | 120 | 120 | 120 |

As can be seen from Table 14, when water is added to the reactants, the yield of acetic acid can be enhanced.

## Claims

1. A process for the selective production of acetic acid, which comprises:
a) pretreating carbon monoxide containing metallic carbonyl compounds by feeding it into a purification column wherein iodine is introduced in a molar amount ranging from 0.1 to 1000 times the metallic content in the feed gas and which is maintained at a temperature to allow the metallic carbonyl impurities to react completely with the iodine gas, sending the metallic iodides formed to an adsorbent column and allowing the metallic iodides to be adsorbed onto the adsorbent therein;
b) carbonylating methanol in a gas phase with said pretreated carbon monoxide in a carbonylation reactor, said carbon monoxide being optionally in admixture of hydrogen, in the presence of a rhodium catalyst comprised of a rhodium compound and a second metallic component selected from the group consisting of an alkali metal, an alkaline earth metal, Co and a mixture thereof, and supported on an inert material, and a halide co-catalyst at a temperature ranging from 200°C to 280°C, a pressure ranging from 10 to 20 atm and a methanol GHSV ranging from 300 to 5000 hr⁻¹ to provide a product mixture composed of acetic acid in a major amount and methyl acetate in a minor amount;
c) separating said methyl acetate in the minor amount and the co-catalyst from the product mixture and recycling the separated methyl acetate and the co-catalyst to the carbonylation reactor;and
d) recovering from the product mixture said acetic acid in the major amount.

2. The process of claim 1, wherein said rhodium compound is selected from the group consisting of RhX₃, RhX₃.3H₂O, Rh₂(CO)₄X₂, [Rh(CO)X₄]Y, Rh₂(CO)₈, Rh(NO₃)₃, [Rh(CO)₂X₂]Y, Rh₂O₃, Rh(CH₃COO)₃, [Rh(C₂H₄)₂X]₂, Rh[(C₆H₅)₃P]₂(CO)X, Rh metal, RhX[(C₆H₅)₃P]₂(CH₃X)₂, Rh(SnX₃)[(C₆H₅)P]₃, RhX(CO)[(C₆H₅)₃Q]₂, (R₄Z)[Rh(CO)₂X)₂, (R₄Z)₂[Rh(CO)X₄], RhX[(C₆H₅)₃P]₃, RhX[(C₆H₅)₃P)H₂, [(C₆H₅)₃P]₃Rh(CO)H and Y₄Rh₂X₂(SnX₃)₄ wherein X is Cl, Br or I; Y is Na, Li or K; Z is N, As or P; Q is As, P or Sb; and R is a C₁ to C₁₂ alkyl or aryl group.

3. The process of claim 1, wherein said alkali metal is selected from the group consisting of Li, Na, K, Rb, Cs and Fr.

4. The process of claim 1, wherein said alkaline earth metal is selected from the group consisting of Be, Mg, Ca, Sr, Ba and Ra.

5. The process of claim 1, wherein said co-catalyst is selected from the group consisting of CH₃I, CH₃Br, CH₃Cl, I₂, Br₂, Cl₂, HI, HBr and HCl.

6. The process of claim 1, wherein said hydrogen is added to said carbon monoxide in an amount of up to 50 mol% based on the amount of said carbon monoxide.

7. The process of claim 1, wherein water is introduced into the carbonylation reactor in an amount of up to 20 mol% based on the amount of said methanol.

## Patentansprüche

1. Verfahren zur selektiven Herstellung von Essigsäure, das umfaßt:
a) Vorbehandlung von Kohlenmonoxid, das metallische Carbonylverbindungen enthält, indem es in eine Reinigungssäule zugeführt wird, in welche Jod in einer molaren Menge eingeführt wird, die im Bereich vom 0,1- bis 1000fachen des Metallgehalts in dem Zuführgas liegt, und welches bei einer Temperatur gehalten wird, um zu ermöglichen, daß die Metallcarbonyl-Verunreinigungen vollständig mit dem Jodgas reagieren, Überführen der gebildeten Metalljodide an eine Adsorptionssäule und Ermöglichen, daß die Metalljodide auf dem Adsorbens darin adsorbiert werden;
b) Carbonylieren von Methanol in einer Gasphase mit dem vorbehandelten Kohlenmonoxid in einem Carbonylierungsreaktor, wobei das Kohlenmonoxid wahlweise in einer Beimischung von Wasserstoff vorliegt, in der Gegenwart eines Rhodiumkatalysators, der eine Rhodiumverbindung und eine zweite metallische Komponente, ausgewählt aus der Gruppe, bestehend aus einem Alkalimetall, einem Erdalkalimetall, Co und einem Gemisch davon, umfaßt und auf einem inerten Material aufgebracht ist, und eines Halogenid-Co-Katalysators bei einer Temperatur im Bereich von 200° C bis 280° C, einem Druck im Bereich von 10 bis 20 atm und einer Methanol-GHSV im Bereich von 300 bis 5000 hr⁻¹, um ein Produktgemisch bereitzustellen, das aus Essigsäure in einer größeren Menge und Methylacetat in einer geringeren Menge zusammengesetzt ist;
c) Trennen des in geringerer Menge vorliegenden Methylacetats und des Co-Katalysators vom Produktgemisch und Rückführen des abgetrennten Methylacetats und des Co-Katalysators zum Carbonylierungsreaktor; und
d) Gewinnen der größeren Menge Essigsäure aus dem Produktgemisch.

2. Verfahren nach Anspruch 1, wobei die Rhodiumverbindung ausgewählt ist aus der Gruppe, bestehend aus RhX₃, RhX₃.3H₂O, Rh₂(CO)₄X₂, [Rh(CO)X₄]Y, Rh₂(CO)₈, Rh(NO₃)₃, [Rh(CO)₂X₂]Y, Rh₂O₃, Rh(CH₃COO)₃, [Rh(C₂H₄)₂X]₂, Rh[(C₆H₅)₃P]₂(CO)X, Rh-Metall, RhX[(C₆H₅)₃P]₂(CH₃X)₂, Rh(SnX₃)[(C₆H₅)P]₃, RhX(CO)[(C₆H₅)₃Q]₂, (R₄Z)[Rh(CO)₂X]₂, (R₄Z)₂[Rh(CO)X₄], RhX[(C₆H₅)₃P]₃, RhX[(C₆H₅)₃P]H₂, [(C₆H₅)₃P]₃Rh(CO)H und Y₄Rh₂X₂(SnX₃)₄ wobei X für Cl, Br oder I steht; Y für Na, Li oder K steht; Z für N, As oder P steht; Q für As, P oder Sb steht; und R für eine C₁- bis C₁₂-Alkyl- oder Aryl-Gruppe steht.

3. Verfahren nach Anspruch 1, wobei das Alkalimetall ausgewählt ist aus der Gruppe, bestehend aus Li, Na, K, Rb, Cs und Fr.

4. Verfahren nach Anspruch 1, wobei das Erdalkalimetall ausgewählt ist aus der Gruppe, bestehend aus Be, Mg, Ca, Sr, Ba und Ra.

5. Verfahren nach Anspruch 1, wobei der Co-Katalysator ausgewählt ist aus der Gruppe, bestehend aus CH₃I, CH₃Br, CH₃Cl, I₂, Br₂, Cl₂, HI, HBr und HCl.

6. Verfahren nach Anspruch 1, wobei der Wasserstoff zum Kohlenmonoxid in einer Menge von bis zu 50 mol%, bezogen auf die Menge des Kohlenmonoxids, hinzugegeben wird.

7. Verfahren nach Anspruch 1, wobei Wasser in den Carbonylierungsreaktor in einer Menge von bis zu 20 mol%, bezogen auf die Menge des Methanols, eingeführt wird.

## Revendications

1. Procédé de production sélective d'acide acétique qui comprend les étapes consistant à :
a) traiter préalablement du monoxyde de carbone contenant des composés de carbonyle métalliques en l'envoyant dans une colonne de purification dans laquelle on introduit de l'iode en une quantité molaire se situant de 0,1 à 1000 fois la teneur métallique dans le gaz d'alimentation et qui est maintenue à une température qui permet aux impuretés de carbonyle métallique de réagir complètement avec le gaz iode, envoyer les iodures métalliques formés vers une colonne adsorbante et permettre aux iodures métalliques d'être adsorbés sur l'adsorbant qui y est contenu ;
b) carbonyler le méthanol en phase gazeuse avec ledit monoxyde de carbone préalablement traité, dans un réacteur de carbonylation, ledit monoxyde de carbone étant éventuellement en mélange avec de l'hydrogène, en présence d'un catalyseur au rhodium constitué d'un composé du rhodium et d'un second composant métallique choisi dans le groupe comprenant un métal alcalin, un métal alcalino-terreux, Co et un de leurs mélanges, et fixé sur un matériau inerte formant support, et d'un co-catalyseur de type halogénure à une température se situant de 200°C à 280°C, sous une pression allant de 10 à 20 atmsophères et avec une VSHG du méthanol se situant de 300 à 5000 h⁻¹, pour fournir un mélange de produits constitué d'acide acétique en quantité importante et d'acétate de méthyle en faible quantité ;
c) séparer ledit acétate de méthyle en faible quantité et le co-catalyseur d'avec le mélange de produits et recycler l'acétate de méthyle séparé et le co-catalyseur vers le réacteur de carbonylation ; et
d) récupérer dans le mélange de produits, ledit acide acétique en quantité importante.

2. Procédé, selon la revendication 1, dans lequel ledit composé du rhodium est choisi dans le groupe comprenant RhX₃, RhX₃.3H₂O, Rh₂(CO)₄X₂, (Rh(CO)X₄]Y, Rh₂(CO)₈, Rh(NO₃)₃, [Rh(CO)₂X₂]Y, Rh₂O₃, Rh(CH₃COO)₃, [Rh(C₂H₄)₂X]₂, Rh[(C₆H₅)₃P)₂(CO)X, métal Rh, RhX[(C₆H₅)₃P]₂-(CH₃X)₂, Rh(SnX₃)[(C₆H₅)P]₃, RhX(CO)[(C₆H₅)₃Q]₂, (R₄Z)[Rh(CO)₂X]₂, (R₄Z)₂[Rh(CO)X₄], RhX[(C₆H₅)₃P]₃, RhX[(C₆H₅)₃P]H₂, [(C₆H₅)₃P]₃Rh(CO)H et Y₄Rh₂X₂(SnX₃)₄ où X représente Cl, Br ou I ; Y représente Na, Li ou K ; Z représente N, As ou P ; Q représente As, P ou Sb ; et R représente un groupe aryle ou alkyle en C₁ à C₁₂.

3. Procédé selon la revendication 1, dans lequel ledit métal alcalin est choisi dans le groupe comprenant Li, Na, K, Rb, Cs et Fr.

4. Procédé selon la revendication 1, dans lequel ledit métal alcalino-terreux est choisi dans le groupe comprenant Be, Mg, Ca, Sr, Ba et Ra.

5. Procédé selon la revendication 1, dans lequel ledit co-catalyseur est choisi dans le groupe comprenant CH₃I, CH₃Br, CH₃Cl, I₂, Br₂, Cl₂, HI, HBr et HCl.

6. Procédé selon la revendication 1, dans lequel on ajoute ledit hydrogène audit monoxyde de carbone en une quantité allant jusqu'à 50 % en moles par rapport à la quantité dudit monoxyde de carbone.

7. Procédé selon la revendication 1, dans lequel on introduit de l'eau dans le réacteur de carbonylation en une quantité allant jusqu'à 20 % en moles par rapport à la quantité dudit méthanol.
